# FASCICULE DE BREVET EUROPEEN

(11) **EP 2 949 599 B1**
(45) Date de publication et mention de la délivrance du brevet: **29.03.2017**
(21) Numéro de dépôt: 15173970.3
(22) Date de dépôt: 16.11.2005
(51) Int. Cl.: B65D 75/34, A61M 15/00

(54) **ENSEMBLE DE RESERVOIR POUR DISPOSITIF DE DISTRIBUTION DE PRODUIT FLUIDE**
BEHÄLTERANORDNUNG FÜR VERTEILUNGSVORRICHTUNG FÜR FLÜSSIGE PRODUKTE
RESERVOIR ASSEMBLY FOR A FLUID PRODUCT DISPENSING DEVICE

(30) Priorité: 16.11.2004 FR 0452641
(43) Date de publication de la demande: 02.12.2015
(62) Demande divisionnaire de: 05819153.7
(73) Titulaire: APTAR France SAS, 27110 Le Neubourg (FR)
(72) Inventeur: FOURMENT, Olivier, 75116 PARIS (FR); BRUNA, Pascal, 76300 SOTTEVILLE LES ROUEN (FR)
(74) Mandataire: CAPRI

(56) Documents cités:
- WO-A-93/15972
- WO-A-2004/011070
- WO-A-2004/041672
- DE-A1- 19 805 336

## Description

La présente invention concerne un ensemble de réservoir pour dispositif de distribution de produit fluide, et plus particulièrement pour un dispositif d'inhalation de produit pulvérulent ou poudre, selon le préambule de la revendication 1.

Les dispositifs d'inhalation de poudre sont bien connus dans l'état de la technique. Certain de ces dispositifs comportent un réservoir commun contenant une pluralité de doses de poudre et des moyens de dosage adaptés, à chaque actionnement, à prélever une dose de poudre destinée à être expulsée vers l'utilisateur. D'autres dispositifs prévoient des réservoirs prédosés individuels (cf. WO 2004/011070 A), par exemple des blisters, contenant chacun une dose unique de poudre, chaque réservoir étant ouvert individuellement lors d'un actionnement pour permettre l'expulsion du produit qu'il contient. Dans le cadre d'un dispositif avec réservoir(s) prédosé(s), différentes solutions ont été proposées pour ouvrir le réservoir et accéder au contenu afin de permettre son expulsion. Ainsi, il a été proposé de réaliser des réservoirs prédosés avec une couche pelable permettant d'exposer le réservoir et sa poudre à un écoulement de gaz, en général de l'air pour l'expulsion de la dose. D'autres dispositifs prévoient des moyens de perçage pour percer ou déchirer une partie du réservoir, permettant ainsi également d'expulser le contenu de celui-ci. Un problème qui peut se poser avec ces dispositifs concerne le risque de perte de doses, partielle ou totale, après ouverture du réservoir et avant inhalation. Ceci diminue la précision de dosage et la reproductibilité de dose (sous-dosage). Un autre problème encore plus important concerne le risque de surdosage. Ceci peut par exemple se produire si, dans un dispositif ayant des réservoirs prédosés, un réservoir est ouvert, mais l'appareil n'est pas utilisé de sorte que, le contenu du réservoir n'est pas inhalé. Dans ce cas, si du produit est susceptible de sortir du réservoir et de se déposer dans des parties du dispositif, lors du prochain actionnement, qui provoquera l'ouverture d'un autre réservoir, le risque de surdosage existe. En effet, le produit sorti du premier réservoir pourra être expulsé ensemble avec la dose complète du second réservoir. Selon la nature du produit, qui peut être un produit pharmaceutique, ce surdosage risque d'être très néfaste à l'utilisateur. Un autre problème qui se pose avec les dispositifs d'inhalation de poudre concerne la précision et la reproductibilité du dosage. En effet, il peut arriver que le vidage du réservoir ou du système de dosage ne doit pas toujours réalisé complètement à chaque actionnement, ce qui engendre des risques de différence de dosage d'une dose à l'autre, et donc une perte de précision et de reproductibilité de dosage.

La présente invention a donc pour but de fournir un ensemble de réservoir pour dispositif de distribution de produit fluide qui ne reproduit pas les inconvénients susmentionnés.

La présente invention a aussi pour but de fournir un dispositif de distribution de produit fluide qui assure une distribution précise, fiable et reproductible à chaque actionnement du dispositif.

La présente invention a encore pour but de fournir un ensemble de réservoir pour dispositif de distribution de produit fluide qui garantit une distribution de la totalité de la dose à chaque actionnement.

La présente invention a aussi pour but de fournir un ensemble de réservoir pour dispositif de distribution de produit fluide qui limite au maximum, voir évite, tout risque de perte de dose après ouverture du réservoir mais avant inhalation, et qui interdit tout risque de surdosage, notamment en cas d'ouverture d'un réservoir prédosé qui ne serait pas suivie d'une inhalation.

La présente invention a encore pour but de fournir un ensemble de réservoir pour dispositif de distribution de produit fluide qui empêche tout risque de contamination du produit à distribuer.

La présente invention a également pour but de fournir un dispositif de distribution de produit fluide qui soit simple et peu coûteux à fabriquer et à assembler.

La présente invention a donc pour objet un ensemble de réservoir tel que défini dans la revendication 1.

Avantageusement, plusieurs réservoirs prédosés sont formés sur un support commun, comportant une couche de base commune pourvue d'une pluralité de cavités et une couche de fermeture commune fermant hermétiquement ladite pluralité de cavités.

D'autres caractéristiques et avantages de la présente invention apparaîtront plus clairement au cours de la description détaillée suivante d'un mode de réalisation avantageux de celle-ci, faite en référence aux dessins joints, donnés à titre d'exemples non limitatifs, et sur lesquels :
- la figure 1 est vue schématique en section transversale d'une partie d'un dispositif de distribution de produit fluide selon un mode de réalisation avantageux, avant actionnement du système d'ouverture de réservoir et avant inhalation de l'utilisateur ;
- la figure 2 est une vue similaire à celle de la figure 1, après actionnement dudit système d'ouverture de réservoir et avant inhalation de l'utilisateur ;
- la figure 3 est une vue similaire à celles des figures 1 et 2, pendant l'inhalation de l'utilisateur ;
- la figure 4 est une vue similaire à celle des figures 1 à 3, après actionnement du système d'ouverture du réservoir et en l'absence d'inhalation subséquente ;
- la figure 5 est une vue schématique en section transversale d'un réservoir prédosé tel que représenté sur la figure 4 ;
- la figure 6 est une vue de dessus du réservoir de la figure 5 ; et
- la figure 7 est une vue de détail d'un élément de perçage du dispositif représenté sur les figures 1 à 4.

L'invention s'applique plus particulièrement aux dispositifs d'inhalation de produit pulvérulent ou poudre, dont un mode de réalisation avantageux sera décrit ci-après. Il est toutefois entendu que la présente invention n'est pas limitée à ce type de dispositif mais concerne de manière générale tout dispositif de distribution de produit pulvérulent.

Le dispositif d'inhalation comporte au moins un réservoir prédosé 10 hermétiquement fermé contenant une dose unique de produit pulvérulent. Ce réservoir 10 comprend avantageusement une couche de base 11 formant une cavité recevant la poudre. Cette couche de base peut être en aluminium. Une couche de fermeture 12 est fixée à ladite couche de base 11, par exemple par collage, thermosoudure, soudure, ou tout autre moyen de fixation approprié. Cette couche de fermeture est étanche à la poudre, à l'air et à l'humidité, et peut comprendre de l'aluminium. Plusieurs réservoirs prédosés peuvent être formés sur un support commun, par exemple sous la forme d'une bande allongée, d'un disque ou d'un cylindre. Dans ce cas, le support commun peut comporter une couche de base commune 11 contenant une pluralité de cavités et une couche de fermeture commune 12, fermant hermétiquement ladite pluralité de cavités. D'autres structures sont aussi envisageables.

Le dispositif comporte en outre un canal d'expulsion 20 qui débouche dans un embout buccal (non représenté). Un système d'ouverture de réservoir 30 est prévu pour permettre d'ouvrir un réservoir prédosé 10 à chaque actionnement du dispositif.

Avantageusement, lorsque le dispositif comporte une pluralité de réservoirs prédosés, le dispositif comporte en outre des moyens de transport (non représentés) qui sont adaptés à amener un réservoir prédosé respectif 10 dans une position de distribution à chaque actionnement du dispositif pour permettre son ouverture par le système d'ouverture de réservoir 30, et l'inhalation du produit qu'il contient à travers le canal d'expulsion 20. Avantageusement, en position de distribution, le réservoir prédosé 10 coopère de manière étanche avec le canal d'expulsion 20. Ces moyens de transports pouvant être quelconque, et n'intervenant pas directement dans l'invention, ils ne seront pas décrits plus en détails ci-après.

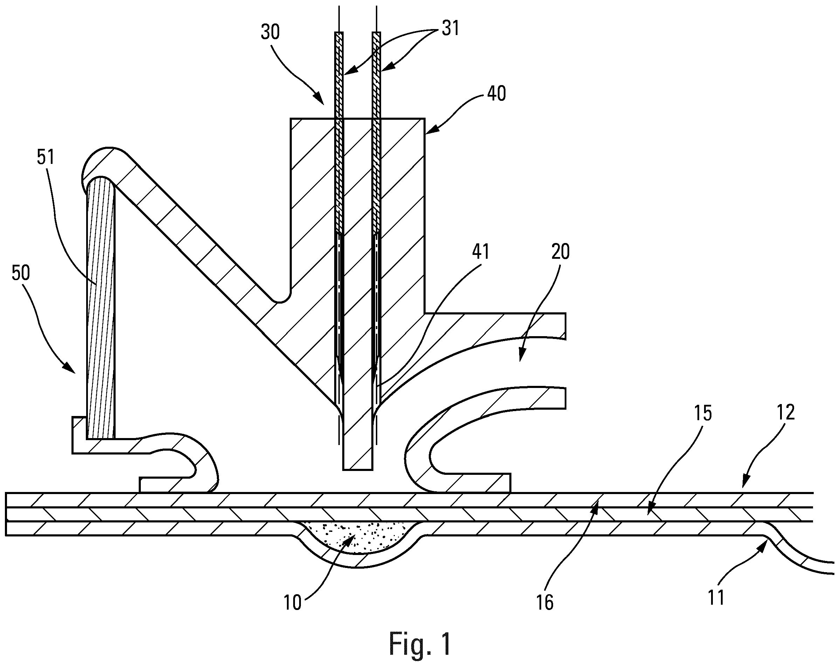

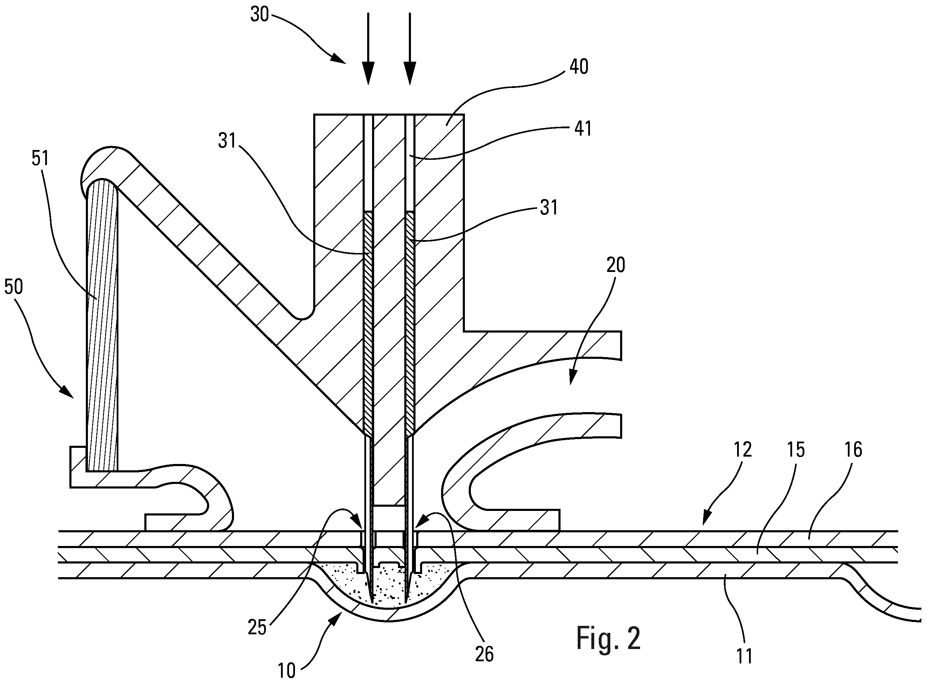

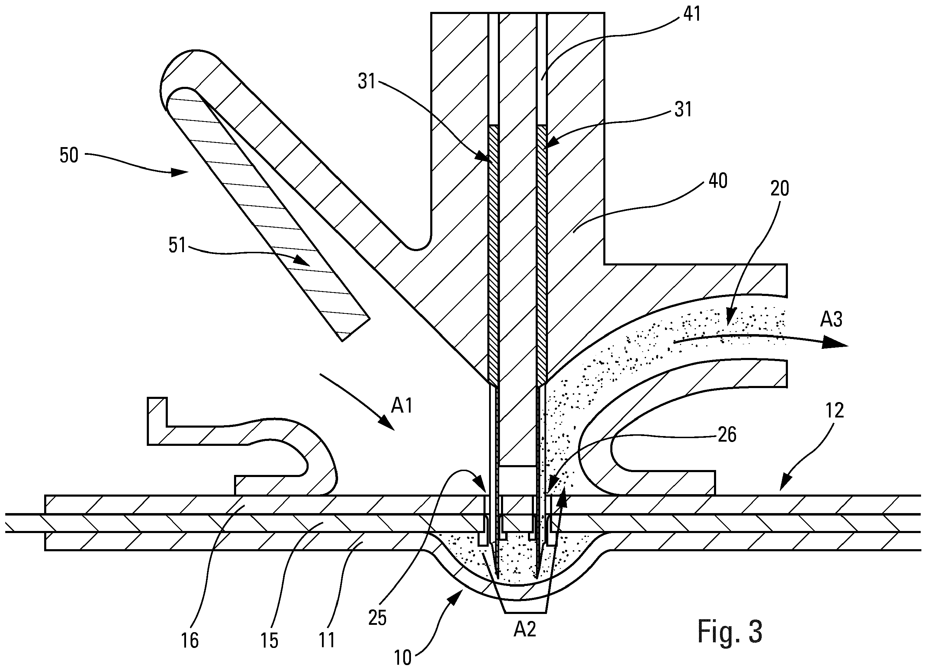

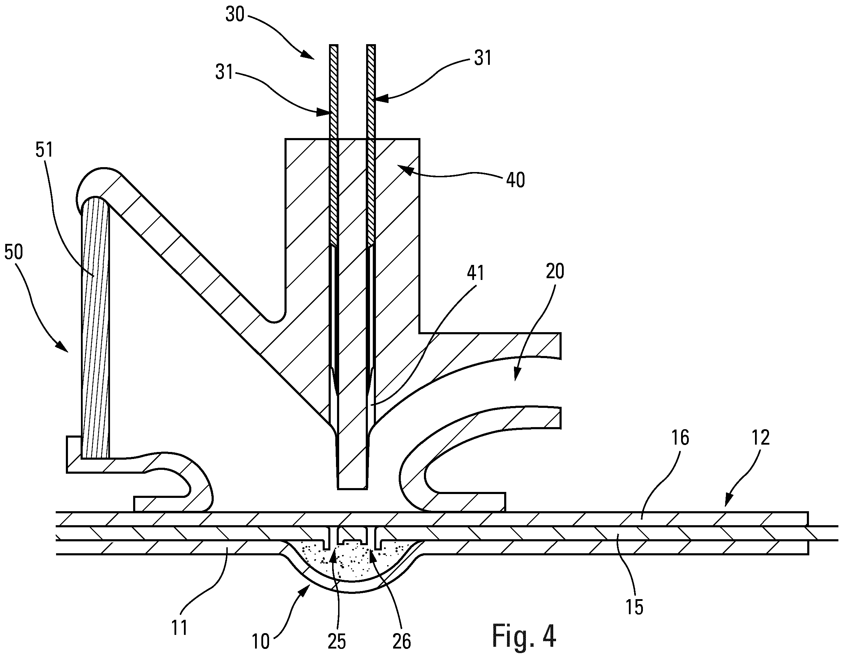

Le système d'ouverture 30 peut comporter au moins deux éléments de perçage 31 qui sont actionnés pour créer au moins deux ouvertures distinctes dans la couche de fermeture 12 du réservoir prédosé 10. La suite de la description sera faite en relation à deux éléments de perçage 31, mais il est entendu qu'il peut y en avoir plusieurs, par exemple trois ou quatre. De préférence, ces éléments de perçage sont déplacés manuellement et simultanément, comme représenté sur les figures 1 à 4. En particulier, ces éléments de perçages 31 peuvent être déplaçables entre une position de repos, représentée sur les figures 1 et 4, et une position de perçage représentée sur les figures 2 et 3, lesdits éléments de perçage étant de préférence en position de perçage au moment de l'expulsion de la dose de produit pulvérulent hors du réservoir prédosé 10. En fait, l'extrémité de perçage de chaque élément de perçage 31 forme de préférence une partie du canal d'expulsion 20 au moment de l'expulsion. Avantageusement, le système d'ouverture 30 est actionné au moyen d'un organe de commande (non représenté) qui peut être un couvercle ou capot de protection du dispositif. Ainsi, les éléments de perçage 31 peuvent être déplacés vers leur position de perçage lors de l'ouverture du capot et ramenés vers leur position de repos lors de la fermeture dudit capot.

En se référant plus précisément aux figures 1 à 4, on constate qu'en position de distribution, le réservoir prédosé 10 coopère avec une partie amont du canal d'expulsion 20 situé en amont du réservoir 10 et une partie aval du canal d'expulsion 20 disposée en aval dudit réservoir. Au moment de l'inhalation, et dans les conditions qui sont décrites ci-après, un écoulement de gaz, notamment d'air, est créé à travers ledit canal d'expulsion 20, l'écoulement de gaz s'écoulant à partir de la partie amont du canal d'expulsion. Cet écoulement peut ensuite pénétrer à l'intérieur du réservoir prédosé 10 à travers au moins une ouverture d'entrée 25 formée par au moins un des éléments de perçage 31. L'écoulement de gaz se mélange alors avec la poudre qui est contenue de ce réservoir et l'ensemble ressort à travers au moins une seconde ouverture de sortie formée par au moins un autre élément de perçage 31, pour ensuite s'écouler dans la partie aval du canal d'expulsion 20 en direction de l'embout buccal (non représenté). Cette séquence est schématiquement représentée sur la figure 3, la flèche A1 montrant l'écoulement de gaz dans la partie amont du canal d'expulsion, la flèche A2 montrant le cheminement de l'écoulement de gaz à l'intérieur du réservoir prédosé 10, et la flèche A3 montrant l'écoulement de gaz et poudre se déplaçant en direction de l'embout buccal.

Le dispositif de distribution comporte également un système de déclenchement par inhalation 50. Ce système de déclenchement par inhalation 50 comporte avantageusement un clapet 51 déplaçable entre une position de fermeture et une position d'inhalation. Dans la position de fermeture représentée sur les figures 1, 2 et 4, le clapet mobile 51 obture la partie amont du canal d'expulsion 20. De préférence, cette obturation est sensiblement étanche. De préférence, le clapet mobile 51 est sollicité vers sa position de fermeture, par tous moyens quelconque, par exemple la gravité, ou des moyens séparés, tel qu'un ressort. Le clapet mobile 51 est adapté à se déplacer vers sa position d'inhalation lorsque l'inhalation de l'utilisateur atteint un seuil prédéterminé. Tant que l'utilisateur n'inhale pas suffisamment fort pour atteindre ce seuil, le clapet mobil 51 reste fermé et aucun écoulement de gaz ne peut se propager à l'intérieur du canal d'expulsion 20, de sorte que le produit ne peut pas être expulsé hors du réservoir 10. Au moment où l'inhalation de l'utilisateur atteint le seuil prédéterminé, le clapet mobile 51 s'ouvre, par exemple pivote comme représenté sur la figure 3, ce qui provoque la création d'un écoulement de gaz, notamment d'air, qui provoquera l'expulsion du produit pulvérulent comme expliqué précédemment.

Ainsi, lors de l'expulsion du produit, les éléments de perçage 31 sont de préférence dans leur position de perçage, dans laquelle ils pénètrent à l'intérieur du réservoir prédosé 10, à travers la couche de fermeture 12. Dans cette position, si l'utilisateur agite ou bouge le dispositif, les risques de perte de produit pulvérulent sont quasiment inexistants. En effet, la forme spécifique des éléments de perçage 31 rend toute sortie de poudre quasiment impossible. On pourrait aussi envisager que le canal d'expulsion comporte une forme spécifiquement adaptée pour éviter d'avantage encore les risques de perte de dose. Par exemple, des déflecteurs ou parties coudées pourraient être prévus qui retiendraient des éventuelles pertes de poudre dans le canal, de sorte que lors de l'inhalation, la totalité de la dose serait distribuée. L'invention permet donc de limiter très fortement le risque d'une perte de dose (sous-dosage) après ouverture du réservoir et avant inhalation.

Au moment de l'inhalation, l'écoulement de gaz a une puissance suffisante pour assurer un vidage total et complet du réservoir prédosé 10, en raison du seuil prédéterminé minimal que l'utilisateur doit atteindre pour provoquer l'ouverture du clapet mobile 51. L'invention permet donc de garantir un vidage complet et total et donc une précision et une reproductibilité de dosage à chaque actionnement.

Avantageusement, le dispositif comporte un corps 40 qui définit au moins partiellement le canal d'expulsion 20. Ce corps 40 peut en outre comprendre des moyens de guidage 41 pour les éléments de perçage 31 du système d'ouverture 30. Avantageusement, ce corps 40 forme également le support de l'élément de clapet mobile 51. Enfin, en position de distribution du réservoir prédosé, ce corps 40 coopère avantageusement de manière étanche avec ledit réservoir garantissant une expulsion fiable, totale, complète et reproductible à chaque actionnement. Cette mise en oeuvre permet de simplifier la fabrication et l'assemblage du dispositif, et donc d'en diminuer le coût de manière non négligeable.

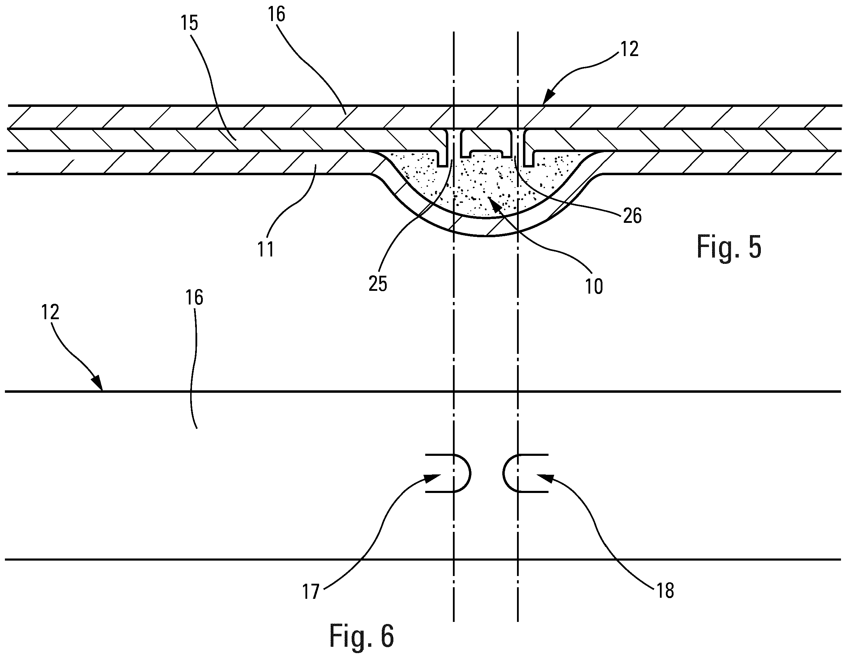

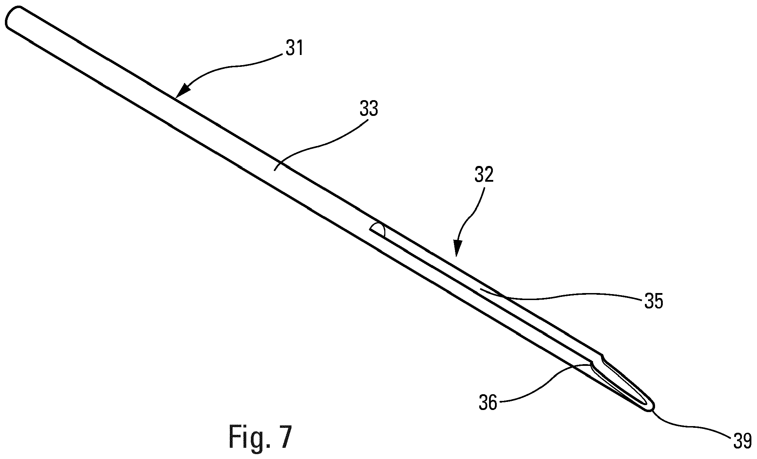

En se référant maintenant plus particulièrement à la figure 7, où il est représenté une vue de détail d'un élément de perçage 31, on constate que cet élément de perçage 31 comporte une partie de corps plein 33 et une extrémité de perçage 32 au moins partiellement creuse. C'est cette extrémité de perçage 32 qui forme une partie du canal d'expulsion 20 en position de perçage. Pour se faire, l'extrémité de perçage 32 est avantageusement réalisée sous la forme d'une pointe creuse dont une partie paroi longitudinale est découpée ou retirée pour former une ouverture longitudinale 35 sur un côté de ladite extrémité. L'exemple représenté sur la figure 7 montre une ouverture 35 formant environ un tiers de la périphérie de la pointe, mais bien entendu cette ouverture pourrait avoir des dimensions supérieures ou inférieures à celles représentées. Par exemple, l'ouverture longitudinale pourraient représentée entre environ 20% et environ 80% de la périphérie de la pointe. Par ailleurs, le bout de l'extrémité de perçage 39 est de préférence effilé, pour permettre de réaliser un perçage net et précis. Avantageusement, la dimension périphérique de l'ouverture longitudinale 35 peut également être modifiée au niveau dudit bout 39, par exemple augmentée progressivement en direction dudit bout. La partie effilée formant le bout 39 de l'extrémité de perçage 32 forme avantageusement une pente inclinée qui permet de réaliser une découpe progressive et précise au fur et à mesure que l'élément de perçage pénètre dans le réservoir 10 à travers la couche de fermeture 12. Avantageusement, un épaulement 36 peut être prévu au niveau de la jonction entre la pente inclinée du bout 39 et un bord de l'ouverture longitudinale 35, afin d'améliorer le basculement de la découpe 17, 18 formée par l'élément de perçage 31 dans la couche de fermeture 12. La figure 6 représente schématiquement une vue de dessus de la couche de fermeture 12, et montre les deux découpes 17, 18 formées par les deux éléments perçage 31. On constate que ces découpes 17, 18 restent solidaires de la couche de fermeture 12 au niveau du côté ouvert 35 de chaque élément de perçage 31. Avantageusement, les éléments de perçage 31 sont identiques, mais des différences pourraient aussi être envisagées entre le ou les élément(s) de perçage formant le ou les passage(s) d'entrée 25, et le ou les élément(s) de perçage formant le ou les passage(s) de sortie 26, par exemple au niveau de certaines dimensions.

Avantageusement, les côtés ouverts 35 des deux éléments perçage 31 sont disposés en éloignement l'un de l'autre, ce qui signifie que les côtés fermés de ces extrémités de perçage 32 se font face. Cette mise en oeuvre améliore le transport de l'écoulement de gaz vers l'intérieur du réservoir ainsi que sa sortie avec la poudre hors de celui-ci. Avantageusement, l'extrémité de perçage 32 s'étend le long de l'élément de perçage 31 sur une distance telle qu'en position de perçage (représentée sur la figure 2) le bord de l'extrémité de perçage 32 opposée au bout 39 coopère avec le corps 40, favorisant ainsi encore d'avantage l'écoulement de gaz.

Le réservoir prédosé 10 comporte une couche de fermeture 12 de structure complexe. Comme décrit précédemment le réservoir 10 est formé par une couche de base 11, par exemple réalisé en aluminium, et qui comporte la ou les cavité(s) contenant la poudre. La couche de fermeture 12 comporte alors une couche interne 15 réalisée en un matériau étanche au produit à l'air et à l'humidité, de préférence de l'aluminium. Cette couche interne 15 est fixée sur la couche base 11 d'une manière quelconque appropriée pour fermer hermétiquement la ou les cavité(s). La couche de fermeture comporte en outre une couche externe 16 fixée à ladite couche interne 15, également d'une manière quelconque appropriée. Cette couche externe 16 est une couche de protection qui est réalisée en un matériau relativement souple et ayant une mémoire de forme, tel qu'un matériau élastomère. L'avantage de cette structure est que lorsque le système d'ouverture est actionné et que les éléments de perçage 31 viennent percer la couche de fermeture 12, des passages sont créés entre le canal d'expulsion 20 et l'intérieur du réservoir 10. Si l'utilisateur ramène le système d'ouverture 30 en position de repos, c'est-à-dire faire ressortir les éléments de perçage 31 hors du réservoir 10 pour les ramener vers leurs positions de repos respectives, la couche de protection externe 16 se referme de manière étanche pour empêcher toute perte de produit hors du réservoir. Ceci peut par exemple se produire si l'utilisateur ouvre le capot de protection de l'inhalateur et le referme sans avoir inhalé. Alors que la couche interne 15 en aluminium reste déchirée et qu'une ouverture est donc susceptible de rester à cet endroit, la couche de protection externe 16 se referme de par son élasticité en obturant ainsi le réservoir 10, de préférence de manière étanche. Ceci est particulièrement avantageux pour éviter les risques de surdosage. En effet, en l'absence de couche de protection externe, dans l'hypothèse où l'utilisateur ouvre le réservoir puis fait ressortir le système d'ouverture sans avoir inhalé, un risque potentiel peut exister que de la poudre contenu dans ce réservoir ouvert se déverse dans le canal d'expulsion, cette poudre étant ensuite distribuée lors du prochain actionnement ensemble avec la dose complète du prochain réservoir prédosé. Ce surdosage, dans le cas de produit pharmaceutique, peut être hautement néfaste, et l'invention permet efficacement et à faible coût d'éviter ce risque. Bien entendu, la présence de la couche de protection externe 16 permet aussi d'éviter tout risque de perte de dose ou sous-dosage de manière particulièrement efficace.

Il est à noter que cette structure particulière de couche de fermeture 12, avec sa couche externe de protection, pourrait être mise en oeuvre indépendamment du dispositif d'inhalation décrit précédemment, et notamment indépendamment du système de déclenchement par l'inhalation 50. De même, une telle couche de protection externe pourrait également être efficace avec des systèmes comportant un nombre quelconque d'élément de perçage, à savoir un seul ou plusieurs.

Il est à noter que bien que l'invention ait été décrite en référence à un mode de réalisation particulier de celle-ci, elle n'est pas limitée aux exemples représentés. Ainsi, la forme du réservoir peut être quelconque. De même, les matériaux décrits ne sont donnés qu'à titre d'exemples, et d'autres matériaux sont envisageables. De plus, l'invention a été décrite en référence à l'utilisation de deux éléments de perçage 31. Il est entendu que trois ou plusieurs de tels éléments de perçage pourraient être utilisés dans le cadre de la présente invention. En particulier, on pourrait envisager un seul élément de perçage pour former l'ouverture d'entrée du réservoir et deux éléments de perçage pour former la sortie. Par ailleurs, les éléments de perçage ne sont pas nécessairement identiques et l'élément de perçage formant l'ouverture de sortie du réservoir pourrait avoir des dimensions différentes, notamment supérieures, à celle formant l'ouverture d'entrée. De plus, la structure du système de déclenchement par l'inhalation peut être quelconque, la forme du clapet 51 n'étant pas limitative.

D'autres modifications sont aussi envisageables sans sortir du cadre de la présente invention tel que défini par les revendications annexées.

## Revendications

1. Ensemble de réservoir comprenant au moins un réservoir prédosé (10) hermétiquement fermé et contenant une dose unique de produit pulvérulent, chaque réservoir prédosé (10) comportant une couche de base (11) formant une cavité et une couche de fermeture (12) fixée à ladite couche de base (11) pour fermer hermétiquement ladite cavité, ladite couche de fermeture (12) comportant une couche interne (15) en matériau étanche au produit, à l'air et à l'humidité, telle qu'une feuille d'aluminium, et une couche externe de protection (16) fixée sur ladite couche interne +(15), **caractérisé en ce que** ladite
couche externe de protection (16) est réalisée en un matériau ayant une mémoire de forme et sensiblement élastique, tel qu'un matériau élastomère, de sorte que si ladite couche de fermeture est percée par un ou des élément(s) de perçage avec formation de découpes (17, 18), et que ce ou ces élément(s) de perçage sont retirés, ladite couche externe (16) se referme de préférence de manière étanche au niveau desdites découpes (17, 18), empêchant toute sortie de produit pulvérulent hors dudit réservoir (10).

2. -Ensemble de réservoir selon la revendication 1, dans lequel plusieurs réservoirs prédosés sont formés sur un support commun, comportant une couche de base commune (11) pourvue d'une pluralité de cavités et une couche de fermeture commune (12) fermant hermétiquement ladite pluralité de cavités.

3. -Dispositif de distribution de produit fluide, **caractérisé en ce qu'**il comprend un ensemble de réservoir selon l'une quelconque des revendications précédentes.

## Patentansprüche

1. Behälterkomplex, aufweisend mindestens einen vordosierten Behälter (10), der hermetisch verschlossen ist und eine einzige Dosis eines pulverförmigen Produktes enthält, wobei jeder vordosierte Behälter (10) eine Basisschicht (11), die einen Hohlraum bildet, und eine Verschlussschicht (12) aufweist, die an der Basisschicht (11) befestigt ist, um den Hohlraum hermetisch abzuschließen, wobei die Verschlussschicht (12) eine interne Schicht (15) aus einem gegenüber dem Produkt, gegenüber Luft und Feuchtigkeit dichten Material, wie eine Aluminiumfolie, und eine externe Schutzschicht (16) aufweist, die auf der internen Schicht (15) befestigt ist, **dadurch gekennzeichnet, dass** die externe Schutzschicht (16) aus einem Material gefertigt ist, das einen Memory-Effekt aufweist und im Wesentlichen elastisch ist, wie einem elastomeren Material, so dass, wenn die Verschlussschicht durch ein oder mehrere Durchdringungselemente unter Bildung von Abschnitten (17, 18) durchdrungen wird und das oder die Durchdringungselemente zurückgezogen werden, sich die externe Schicht (16) vorzugsweise dichtend an den Abschnitten (17, 18) schließt, wodurch ein Austreten von pulverförmigem Produkt aus dem Behälter (10) verhindert wird.

2. Behälterkomplex nach Anspruch 1, wobei mehrere vordosierte Behälter auf einem gemeinsamen Träger gebildet sind, aufweisend eine gemeinsame Basisschicht (11), die mit mehreren Hohlräumen versehen ist, und eine gemeinsame Verschlussschicht (12), die die mehreren Hohlräume hermetisch verschließt.

3. Ausgabevorrichtung für ein fluides Produkt, **dadurch gekennzeichnet, dass** es einen Behälterkomplex nach einem der vorhergehenden Ansprüche aufweist.

## Claims

1. A reservoir kit comprising at least one predosed reservoir (10) that is hermetically sealed and that contains a single dose of powder, each predosed reservoir (10) comprising a base layer (11) forming a cavity, and a closure layer (12) that is bonded to said base layer (11) so as to seal said cavity hermetically, said closure layer (12) comprising an inner layer (15) that is made of a material that is powder-tight, airtight, and moisture-tight, such as a sheet of aluminum, and a protective outer layer (16) that is bonded on said inner layer (15), the reservoir kit being **characterized in that** said protective outer layer (16) is made of a material that has shape memory and that is substantially elastic, such as an elastomer material, so that if said closure layer is pierced by one or more piercer elements cutting out flaps (17, 18), and the piercer element(s) are then removed, said outer layer (16) closes preferably in sealed manner at said flaps (17, 18), preventing any powder from escaping from said reservoir (10).

2. A reservoir kit according to claim 1, in which a plurality of predosed reservoirs are formed on a single support that comprises a single base layer (11) provided with a plurality of cavities, and a single closure layer (12) that hermetically seals said plurality of cavities.

3. A fluid dispenser device **characterized in that** it comprises a reservoir kit according to any preceding claim.
